# EUROPEAN PATENT APPLICATION

(11) **EP 2 455 449 A1**
(43) Date of publication of application: **23.05.2012**
(21) Application number: 11166842.2
(22) Date of filing: 20.05.2011
(51) Int. Cl.: C12N 1/15, C12N 9/10, A01N 63/04

(54) **Trichoderma atroviride strain with improved biological activity, especially for use in agriculture as a biofungicide and the method of its obtaining**

(30) Priority: 23.11.2010 PL 39102110
(71) Applicant: Instytut Biochemii I Biofizyki Pan, 02-106 Warszawa (PL)
(72) Inventor: Kruszewska, Joanna S., 03-409 Warszawa (PL); Zembek, Patrycja, 05-300 Minsk Mazowiecki (PL); Mach, Robert L., 3011 Untertullnerbach (AT); Perlinska-Lenart, Urszula, 02-570 Warszawa (PL); Brunner, Kurt, 1130 Vienna (AT); Reithner, Barbara, 1160 Vienna (AT); Palamarczyk, Grazyna, 02-350 Warszawa (PL)
(74) Representative: Brodowska, Iwona

(57) **Abstract**

The invention applies to a new *Trichoderma atroviride* strain with improved biocontrol activity, for use in agriculture as a biofungicide. The method of obtaining the *Trichoderma atroviride* strain with elevated biocontrol activity, according to the invention, is performed by modification of the amount of secretory proteins and of their secretion achieved by expression of the *DPM1* gene from *S. cerevisiae* coding for DPM synthase, an enzyme synthesizing Dol-P-Mannose. Expression is obtained by transformation of the fungus with a vector carrying the *DPM1* gene.

The observed enhanced antifungal properties are a result of the *DPM1* gene overexpression leading to elevated cellulolytic activity of secretory proteins and their increased amount.

## Description

The subject of the invention is a new strain of the fungus *Trichoderma atroviride* showing elevated biocontrol activity, for use in agriculture. The increase of the biocontrol activity, according to the invention, is obtained by overexpression of *DPM1* gene resulting in activation of the protein O-glycosylation pathway.

*Trichoderma* belongs to the *Ascomycota* division, *Hypocreales* order and *Hypocreaceae* family. Strains of *Trichoderma* have been isolated from natural habitats, such as from rotting wood and soil. Many of them parasitize other fungi. *Trichoderma* is also found among micorrhizal fungi of tree root system.

### Trichoderma as a biocontrol organism with biofungicide activity

The mechanism of biocontrol is very complicated and the final effect results from a synergistic activity of several processes. Biocontrol is a result of competition for nutrients between *Trichoderma* and pathogenic fungi, but is also an effect of the action of fungistatic metabolites and antibiotics produced by *Trichoderma* on the germination of pathogen spores and/or pathogen growth, and of the modification of soil pH limiting growth of the pathogens.

Another, biocontrol mechanism is through direct interaction of *Trichoderma* with the pathogen (mycoparasitism), including direct contact of both fungi and synthesis of hydrolytic enzymes and toxins. At the same time, *Trichoderma* promotes growth of plants and stimulates plant defense mechanisms (Harmanet al. 2004, Hjeljord i Tronsmo 1998, Yedidia et. al. 1999).

Mycoparasitism is a direct attack of a fungus on another one. This complicated process includes recognition of the pathogenic fungus, penetration of its cells, and extermination.

*Trichoderma* can directly attack numerous species of plant-pathogenic fungi. Recognition of a plant pathogen by *Trichoderma* stimulates in the latter expression of chitinases, glucanases and proteases (Inglis and Kawchuk 2002, Sanz et al. 2004). The type and the amount of secreted enzymes is characteristic for the strain of *Trichoderma.* It is believed that *Trichoderma* constitutively secretes a small amount of chitinases and their production and secretion is induced by monosaccharides cleaved out from the pathogen cell wall polysaccharides. During direct attack *Trichoderma* hyphae tightly surrounded the mycelium of the pathogen to produce the so called appressorium structure, then they secrete hydrolytic enzymes that lyse the cell wall of the pathogen, and the products of hydrolysis are used by *Trichoderma* as nutrients. Appressorium formation, production and secretion of hydrolases and antibiotics are controlled by cAMP and a cascade of MAP kinases (McIntyre et al. 2004).

In 1983 Elad et al. showed that *Trichoderma* senses the presence of another fungus, which brought about a specific growth of *Trichoderma* mycelia toward this fungus. The mechanism of this phenomenon is not known but mutual recognition of the partners and specificity of the antagonistic action against individual pathogens plays a key role in biocontrol. It is known that in the recognition process lectins and glycoproteins are engaged. For example it is believed that the presence of agglutinin bound to beta-1-3-glucan on the surface of *Sclerotium rolfsii* cells is engaged in the specific interaction with *Trichoderma* spores. Using specific antibodies Inbar and Chet (1995) proved that the lectin located along the mycelium of *Sclerotium* adsorbed exclusively spores of *T. hamatum* T-244.

Cell wall degradation requires activity of chitinases, glucanases and proteases. Since the cell wall of *Rhizoctonia solani* and *Sclerotium rolfsii* contains chitin, a biocontrol *T. harzianum* strain secrets a large amount of chitinases and beta 1-3 glucanase which leads to cell wall degradation. The type of hydrolytic enzymes secreted by a biocontrol agent depends on the pathogen recognized: when attacking *Rhizoctonia solani T.harzianum* secrets endochitinases CHIT52, CHIT42 and CHIT33 or CHIT102 with N-acetylglucosaminidase activity, while in the presence of *Sclerotium rolfsii* it only secrets enzymes with exochitinases activity and two acetylglucosaminidases CHIT102 and CHIT73, giving lower mycoparasitic activity (Elad et al. 1982, Haran et al. 1996).

In view of the above, engineering new transgenic biocontrol *Trichoderma* strains seemed a promising approach improving their biocontrol activity and broadening specificity.

Such studies concentrate on overexpression of genes coding for hydrolytic enzymes such as chitinases, glucanases, proteases and cellulases in *Trichoderma.* The mechanism of induction of chitinases upon contact with a pathogen is not elucidated. For example, expression of *ech42* and *chit33* was detected only during direct contact of *T.harzianum* with *Rhizoctonia solani,* while expression of chit36Y did not require the contact of these fungi.

To increase the biocontrol activity of *Trichoderma* several genes coding for chitinases have been overexpressed, however, the chitinase of 42 kDa molecular weight seems to be the most promising. Overexpression of *ech42* gene in *T. virens* significantly improved the biocontrol activity of the strain against *Rhizoctonia solani* compared with the control strain. Promising results have also been obtained for overexpression of *chit33* in *T*. *harzianum* (Dana et al.. 2001, Carsolio et al. 1999, Kullnig et al. 2000).

Overexpression of beta-1,3-glucanases in *T. harzianum* T28 improved the biocontrol properties of this strain against fungi from class Oomycetes whose cell wall contains cellulose and glucans (*Phytophthora*), as well as against those with the cell wall containing chitin (*Rhizoctonia* and *Botrytis*). The cellulolytic activity was also increased in the *T*. *harzianum* T3 strain giving a more effective strain against *Phytophthora ultimum,* a pathogen of cucumbers.

Overexpression of a serine protease (tvsp1) gave a *T. virens* strain of enhanced activity against *Rhizoctonia* but also against *Oomycetes* (Dunne et al. 2000).

There were also similar attempts that failed. *T. harzianum* strain T-35 carrying ChiA gene encoding chitinase from *Serratia marcescens* secreted high amount of the foreign chitinase but has lost ability to produce its own enzymes in the amount found in the parental strain (Haran et al. 1993).

Recently, application of a mixture of *Trichoderma* transformants overexpressing a variety of hydrolytic enzymes has become a popular approach to increasing biocontrol activity.

Our own studies showed that secretion of hydrolases could be stimulated in *Trichoderma atroviride* P1 indirectly by activation of the protein O-glycosylation process.

Our earlier studies revealed a positive correlation between protein O-glycosylation and secretion in *Trichoderma* (Kubicek et al. 1987, Kruszewska et al. 1999). Inhibition of O-glycosylation of secretory proteins blocked totally their secretion to the cultivation medium.

During our experiments we noticed that choline or Tween 80 added to the cultivation medium increase secretion of enzymatic proteins and stimulate activity of dolichyl phosphate mannose (DPM) synthase, a key enzyme of the O-glycosylation process. We also found that *Trichoderma* strains with high protein secretion also had more active DPM synthase.

The new strain of *Trichoderma atroviride* of improved biocontrol activity, for use in agriculture as a biofungicide, according to the invention, overexpresses the *DPM1* gene from the yeast *Saccharomyces cerevisiae.*

The method used to obtain the *Trichoderma atroviride* P1 strain with improved biocontrol activity, due to increased secretion of hydrolases by this fungus, according to the invention, relies on activation of the O-glycosylation process by integration of the yeast *DPM1* gene into the *Trichoderma* genome by means of genetic transformation.

According to the invention, modification of the level of protein secretion by *Trichoderma* and their activity is obtained by introduction of the *DPM1* gene into the *Trichoderma* genome by transformation through amplification of the gene, its cloning in an appropriate vector and introduction to the fungal cells where the gene is integrated to the genomic DNA and transcribed giving *DPM1* mRNA serving as a template for synthesis of the enzymatic protein and in that way increasing the total activity of DPM synthase.

Unexpectedly, it turned out that while the activity of DPM synthase in *T. reesei* was doubled by overexpression of the yeast *DPM1* gene coding for this enzyme, the overall protein secretion was seven fold higher than in the untransformed strain. Transformation of this *Trichoderma* strain with the *DPM1* gene greatly stimulated the protein glycosylation activity of the transformants. Although the transformants secreted much more proteins, they were still glycosylated to the same extent as in the control. Western blotting confirmed higher secretion of cellulases, among them CBHI. A study of *cbh1* gene expression in the transformants revealed no changes at the transcription level compared to the control. These results together with the results of Western blotting allowed us to conclude that regulation of CBHI expression occurred at the translation and/or posttranslational modification levels. This observation pointed at the essential role of efficient O-glycosylation in the synthesis and processing of proteins up to their release from the cell. The more efficient O-glycosylation of synthesized proteins enables their more efficient transfer through the endoplasmic reticulum, Golgi Ap to the secretory vesicles and finally secretion.

In the method, according to the invention, before introduction of the gene into the *Trichoderma* genome, the *DPM1* gene from *Saccharomyces cerevisiae* is amplified using PCR method and DPM1s (pattern 1) and DPM1r (pattern 2) primers. The PCR method enables a DNA fragment to be obtained with suitable ends necessary for insertion into the expression vector. The fragment is amplified devoid of the original ATP codon for translation start; the ATG codon is present in the cloning site in the expression vector.

In the method, according to the invention, the gene is cloned under the *gpdA* (glyceraldehyde-3-phosphate dehydrogenase) promoter and *trpC* (indole-3-glycerol-phosphate synthase) terminator into the pAN521 N vector. The chosen promoter and terminator, originate from the filamentous fungus *Aspergillus* closely related to *Trichoderma.* To obtain expression the gene should be cloned under the promoter easily recognized by the host. In this respect filamentous fungi are rather un-demanding, being able to recognize promoters from other, not so closely fungi, for example yeast ones.

Profitably, the pAN521 N vector is digested with BamHI restriction endonuclease, the sticky ends are blunted using Mung Bean or S1 nucleases, and the *DPM1* PCR fragment is ligated using T₄ligase.

In the method, according to the invention, to transform *T. atroviride* cotransformation with a mixture of two vectors is performed: the pAN521 N vector carrying the *DPM1* gene and pRMLex_30 bearing a marker gene conferring resistance to Hygromycin B.

Application of the pAN521 N vector requires the use of BamHl restriction enzyme for digestion and the obtained sticky ends have to be blunted by Mung Bean or S1 nucleases to avoid shifting of the Open Reading Frame of the *DPM1* gene.

For the *DPM1* expression some other promoters could be used, for example *cbh1* promoter from gene encoding cellobiohydrolase I or *pki1* promoter from gene encoding pyruvate kinase and other vectors (pAL3, pAL4 or pLMRS3), and for cotransformation a mixture of pAL and p3SR2 vectors could be used (p3SR2 vector contains *amdS* gene encoding aminidase from *A. nidulans*).

The vectors that could be successfully used for transformation include, for example, pAL3 or pAL4 carrying the *alcA* promoter (from a gene encoding alcohol dehydrogenase from *Aspergillus nidulans*).

The pAL3 and pAL4 vectors contain a few original restriction sites (Kpnl, Smal BamHI, Xbal Pstl) in the multiple cloning site and the *pyr4* marker gene enabling selection for uridin auxotrophy.

In this method, according to the invention, *T. atroviride P1* is used as the host for transformation, and this strain is sensitive to Hygromycin B the best marker and this attribute determines the method for selection of transformants.

To obtain blunt ends in the pAN521 N vector, profitably, Mung Bean nuclease is used and it could be replaced by S1 nuclease.

To obtain transformants a cotransformation method was used which means that a mixture of two vectors is used, one carrying the gene encoding the protein which we want to express and the second one, the helper vector, carrying marker gene. In this case, the first vector contains the *DPM1* gene and the second one carries Hygromycin B resistance marker gene.

The two vectors, are used in a ratio of 10 - 15 parts of the first vector and one part of the helper one. It is known that a mixture of vectors used for transformation facilitates uptake of DNA into the cells of filamentous fungi.

Transformants are, selected on plates with a medium containing Hygromycin B and 0.1 % Triton X-100.

The selection medium should contain Hygromycin B to allow selection of transformants insensitive to Hygromycin B which will grow on this medium, whereas Triton X-100 prevents overgrowth of the plate and enables selection of individual transformants.

Next, transformants insensitive to Hygromycin B are checked for the presence of the *DPM1* gene. To this end, DNA is obtained from the transformants and on the template of this DNA a DNA fragment is amplified using primers specific for the *DPM1* gene. Next, the obtained PCR products are analyzed on an agarose gel and bands of about 800 bp are isolated and sequenced. Simultaneously, two control PCR reactions are performed: a positive control reaction on the template of *S.cerevisiae* genomic DNA, and a negative control using *T. atroviride P1* (host strain - untransformed) DNA as the template. Sequences obtained from the sequencing analysis are compared with the known *DPM1* nucleotide sequence.

DNA from the transformants could also be analyzed by the Southern blotting method, but such an analysis takes more time and is more expensive. When Southern blotting is used DNA is digested, with EcoRI, subjected to electrophoresis on agarose gel, denatured, transferred to a nylon membrane and hybridized with a radioactive probe synthesized on the template of the *DPM1* gene (PCR product as above).

The *Trichoderma* genomic DNA should be digested with a restriction enzyme which does not cut inside the *DPM1* gene, for BamHI, ClaI, KpnI, NcoI, NotI, PstI, SphI, or XbaI.

Transformants carrying the *DPM1* gene were also analyzed by the Northern blotting method. For this analysis total RNA was obtained and it was shownthat the transformants contained a *DPM1* transcript. The transformants obtained and verified in the descibed manner were named PZ1/06, PZ6/06 and PZ19/06 and were used for further investigations.

As it was mentioned before the *DPM1* gene encodes DPM synthase, an enzyme which synthesizes dolichyl phosphate mannose (Dol-P-Man). Dol-P-Man serves as a substrate in protein O- and N-glycosylation and in the synthesis of GPI anchor anchoring some proteins in the cell wall and in cell membranes.

### Examples of how to perform the invention

**Example I.** The *DPM1* gene was amplified on the template of *S. cerevisiae* genomic DNA by PCR using DPM1s and DPM1r primers and Expand High Fidelity PCR System (Boehringer Mannheim). The PCR method (Polymerase Chain Reaction) consists in amplification of a DNA fragment using oligonucleotide primers homologous to both ends of the DNA stretch to be amplified and using cyclic heating and cooling when primers are cyclically bound to the template DNA and a new nucleotide chain is synthesized between the primers. During the following temperature cycles the newly synthesized DNA becomes a template for further synthesis. The *DPM1* gene obtained in this way was cloned under the *gpdA* (glyceraldehyde-3-phosphate dehydrogenase) promoter and *trpC* (indole-3-glycerol-phosphate synthase) terminator in the pAN521 N vector (NCBI Accession number Z32697). The vector was digested with BamHI restriction endonuclease, the sticky ends were blunted with Mung Bean nuclease (Promega) and the *DPM1* PCR product was ligated into the vector using T₄ ligase (Promega). The resulting plasmid was used to transform *T.atroviride* P1 strain. The transformation was performed according to Kubicek-Pranz, E.M., Gruber, F., Kubicek, C.P. (1991) Transformation of Trichoderma reesei with the cellobiohydrolase II gene as a means for obtaining strains with increased cellulase production and specific activity. J. Biotechnol. 20, 83-94, and Mach, R.L., Schindler, M., Kubicek, C.P. (1994) Transformation of Trichoderma reesei based on Hygromycin B resistance using homologous expression signals. Curr. Genet. 25, 567-570.

Transformants were selected on medium containing Hygromycin B and 0.1 % Triton X-100. Transformants growing in the presence of Hygromycin B were checked for the presence of *DPM1* gene integrated into their genomic DNA. To this end, genomic DNA was obtained from the transformants using Wizard Genomic DNA Purification Kit (Promega). On the templates of the DNA from the transformants a fragment of DNA was amplified using primers DPM1s and DPM1r. Next, the PCR products were analyzed on agarose gel and bands of about 800 bp were isolated and sequenced to confirm the presence of yeast *DPM1* DNA in the genome of the transformants. In parallel, total RNA was isolated from the transformants and expression of the *DPM1* gene was confirmed by dot-blot Northern analysis showing the presence of *DPM1* mRNA.

Transformants obtained and verified as above were named PZ1/06, PZ6/06 and PZ19/06 and were used for further investigations.

Overexpression of the *DPM1* gene in *T.atroviride* obtained according to the innovation increased the activity of DPM synthase (Table 1).

**Table 1**

| **Effect of overexpression of the *DPM1* gene coding for DPM synthase** | |
|---|---|
| strains | DPM synthase activity [pmol/mg protein/5min] |
| control P1 | 194 ± 15 |
| transformant PZ1/06 | 386 ± 16 |
| transformant PZ6/06 | 318 ± 33 |
| transformant PZ19/06 | 312 ± 17 |

Overexpression of the *DPM1* gene in *T. atroviride* caused an increase of the DPM synthase activity in the membrane fraction obtained from all the transformants. The most pronounced increase, to 199% of the control DPM synthase activity, was noticed for the PZ1/06 strain.

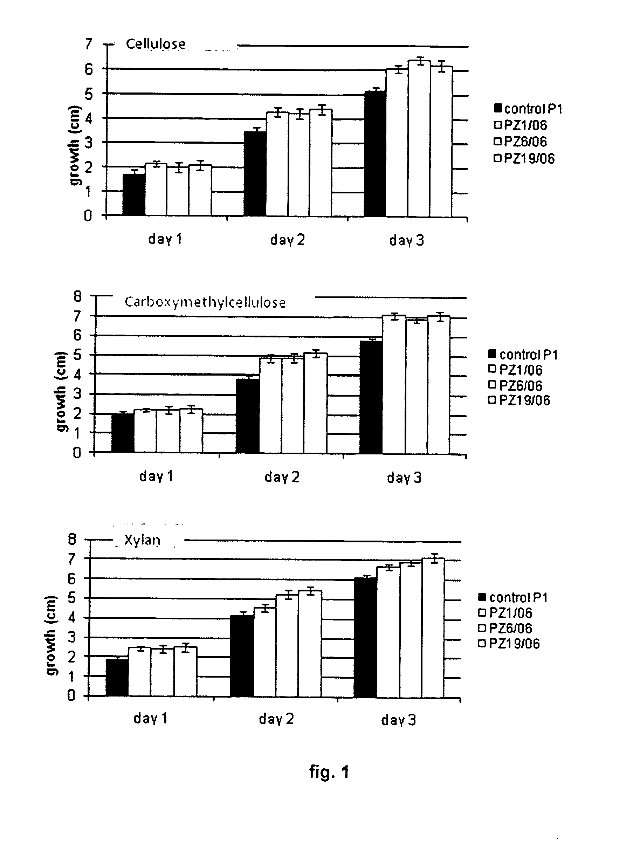

**Example II.** For simple screening of the hydrolytic properties of the obtained strains their growth was examined on plates with MM medium supplemented with different polycarbohydrates such as xylan, cellulose or carboxymethylcellulose as carbon sources. The area of growth was measured for three days and compared with that of the control strain (Fig. 1). All the transformants grew faster than the control strain suggesting that their higher hydrolytic activity enabled more efficient utilization of the complex carbon sources.

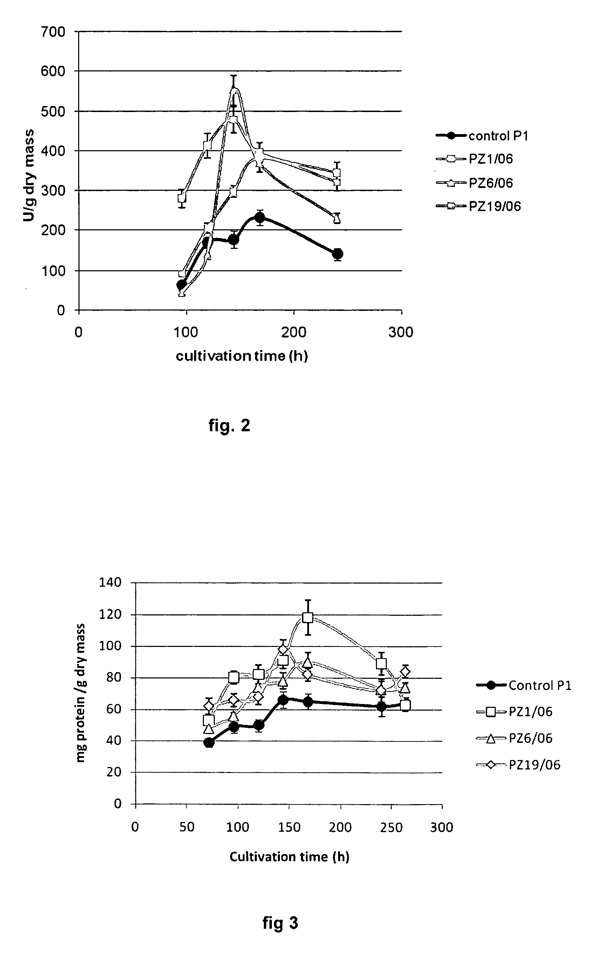

**Example III.** To check whether the transformants secreted higher amounts of cellulases or the cellulases were more active, at first the cellulolytic activity of proteins secreted to the cultivation medium was measured (Fig. 2). It was shown that cellulases secreted by the transformants exhibited higher activity compared to the cellulases secreted by the control P1 strain.

**Example IV.** Next, the amount of proteins secreted to the cultivation medium was measured. The results obtained for transformants (PZ1/06, PZ6/06, PZ19/06) and for the control strain P1 are shown in Fig. 3. All three transformants secreted more protein than did the control strain.

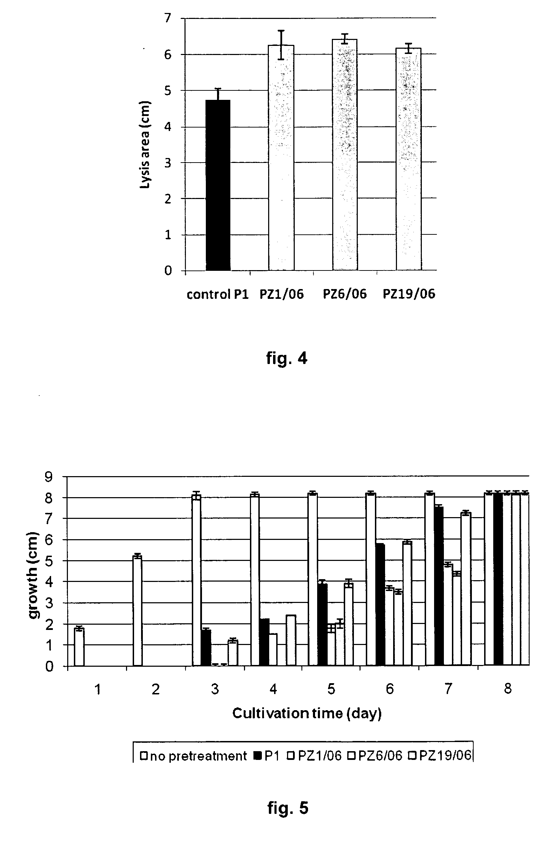

**Example V.** The more efficient utilization of cellulose by the transformants suggested that they could have improved antifungal properties. To verify this assumption, confrontation assay was performed using the new *Trichoderma* strains and the pathogenic fungus *Pythium ultimum* (Fig.4). *Trichoderma* and *Pythium* were cultivated on a single plate from separate inocula and it was observed that *Pythium* was overgrown more rapidly by all the transformants than by the control P1 strain. The experiment was done on minimal medium (SM).

Fig 4. Plate confrontation assay of *T. atroviride* against *P. ultimum.* Mycelial disks of *Trichoderma* transformants PZ1/06, PZ6/06, PZ19/06 and the control strain, and of P. *ultimum* were placed at opposite sides of agar plates and incubated at 28°C for three days.

**Example VI.** It is known that the presence of one fungal species (e.g., the pathogen *Pythium*) may induce in another species (e.g., the biocontrol agent *Trichoderma*) a complex chain of metabolic responses. Such responses may vary in different *Trichoderma* strains. To avoid this complication we sought to study the antifungal properties of the transformants without subjecting them to the presence of the pathogen. To this end, *Trichoderma* transformants and the control strain were cultivated on plates covered with cellophane. After the plates had been grown over, the cellophane with the *Trichoderma* was removed and the plates were used for cultivation of the plant pathogens *P. ultimum* or *R. solani.* In this manner we could study the influence of metabolites secreted by *Trichoderma* on the growth of the pathogens. A negative control comprised plates not subjected to *Trichoderma* culturing. *P. ultimum* failed to grow on the pretreated plates regardless of the *Trichoderma* strain grown on the plates before, while the growth of *R. solani* was strongly inhibited by PZ1/06 and PZ6/06, and slightly by PZ19/06 and the control *Trichoderma* strain (Fig.5). **Example VII.** Since the confrontation experiments revealed enhanced antifungal properties of the transformed *Trichoderma* strains proper biocontrol experiments were performed using the bean *Phaseolus vulgaris* L.

Bean seeds (*Phaseolus vulgaris* L. (var. *nanus* L.)) were coated with 5 x 10⁸ spores of *Trichoderma* per 10 g of seeds. Pathogen-infected soil was prepared by adding 2.2 g of fungal biomass of *Pythium ultimum* to 1 I of sterile soil. Beans were allowed to germinate in pathogen-infected soil for ten days and the number and height of the seedlings was investigated.

Infection by *P. ultimum* brought about an 85% inhibition of plant growth compared with non-infected plants. Infected plants protected by non-transformed *T.atroviride* P1 were by only 37% smaller than the non-infected controls, while protection by the transformed *Trichoderma* strains was even better. In the presence of PZ1/06 and PZ6/06 the inhibition of seedling growth by *P. ultimum* was strongly diminished, to 15 and 10%, respectively, while PZ19/06 not only completely nullified the inhibitory effect of the fungal pathogen, but even caused the seedlings to grow better than the control without any fungus.

Protective effect of *Trichoderma* on bean seedling growth in *P. ultimum*-infected soil. Average size of seedlings measured as seedling height (cm) was analyzed after ten days. Seeds were sown in sterile (black bars) or *P. ultimum*-infected soil (gray bars) in the absence or presence of *Trichoderma* strains as indicated. Ctrl - seeds grown in sterile soil without any fungus.

### Example VIII.

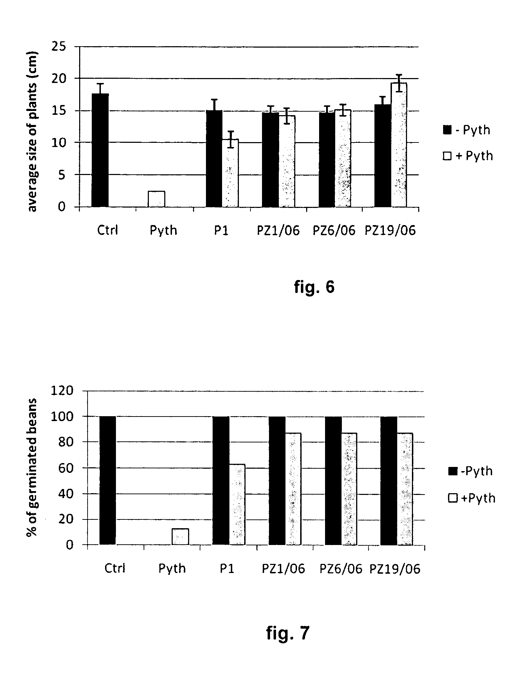

Survival of bean seedlings in soil infected by *P. ultimum* was reduced to 12.5% of control values in sterile soil (Fig.6). Treatment of the seeds with any of the *Trichoderma* strains improved their germination substantially. Each of the transformed *Trichoderma* strains increased the germination rate to 87.5% of the control, and the control P1 strain to 63%.

Protective effect of *Trichoderma* on bean germination in *P. ultimum*-infected soil.

Efficiency of germination presented as percentage of seeds sown was analyzed after ten days.

Seeds were sown in sterile (black bars) or *P. ultimum*-infected soil (gray bars) in the absence or presence of *Trichoderma* strains as indicated. Ctrl - seeds grown in sterile soil without any fungus.

The most spectacular effect of this invention is an unexpected over two-fold increase of cellulolytic activity in the transformants as calculated per dry weight of mycelia, and a significant increase of biocontrol activity compared to the control P1 strain.

The improved biocontrol effect is not connected with any additional costs for cultivation of the transformants and does not require any additional manipulations.

5’ AGC ATC GAA TAC TCT GTT ATC GTT 3’

pattern 1

5’ TTA AAA GAC CAA ATG GTA TAG CTG 3’

pattern 2

## Claims

1. New *Trichoderma atroviride* strain with higher biocontrol activity overexpressing of the *DPM1* gene from *Saccharomyces cerevisiae,* for use in agriculture as a biofungicide.

2. The method of obtaining the *Trichoderma atroviride* strain with elevated biocontrol activity, for use in agriculture as a biofungicide, characterized with enhanced protein O-glycosylation, obtained by integration of the *DPM1* gene from *Saccharomyces cerevisiae* into the *Trichoderma* genome.

3. The method according to the invention, is performed by modification of activity of hydrolytic enzymes and their secretion resulting from integration of the *DPM1* gene into the *Trichoderma* genome, done by amplification of the gene, cloning in the proper vector and transformation into the fungal cells where after integration into the genomic DNA the gene is transcribed giving *DPM1* mRNA serving as a template for synthesis of the enzymatic protein and in that way increasing total DPM synthase activity.

4. The method, according to the patent reservation no. 1, of amplification of the *DPM1* gene from *Saccharomyces cerevisiae* using PCR procedure and DPM1s and DPM1r primers.

5. The method, according to the patent reservation no. 1, of cloning of the *DPM1* gene under the *gpdA* promoter (glyceraldehyde-3-phosphate dehydrogenase) and *trpC* terminator (indole-3-glycerol-phosphate synthase) into the pAN521 N vector, which is cut with BamHI restrictionendonuclease, the sticky ends are blunted by Mung Bean or S1 nuclease, and the *DPM1* fragment (PCR product) is ligated using T₄ ligase.

6. The method according to the patent reservation no. 1, of transformation of *Trichoderma atroviride* using recombinant pAN521 N vector.

7. The method according to the patent reservation no. 1 of selection of the transformants on medium containing Hygromycin B and 0.1 % Triton X-100, and following with the transformants growing on the selective medium are assayed (using PCR procedure or Southern or Northern hybridization) for the presence of integrated *DPM1* gene.
